# EUROPEAN PATENT APPLICATION

(11) **EP 3 053 915 A1**
(43) Date of publication of application: **10.08.2016**
(21) Application number: 15154205.7
(22) Date of filing: 06.02.2015
(51) Int. Cl.: C07D 251/60, C07C 273/12

(54) **METHOD FOR REVAMPING A HIGH PRESSURE MELAMINE PLANT**

(71) Applicant: Casale SA, 6900 Lugano (CH)
(72) Inventor: di Carlo, Gabriele, 6900 Lugano (CH); Rizzi, Enrico, 22070 Casnate con Bernate (CO) (IT)
(74) Representative: Zardi, Marco

(57) **Abstract**

Method for revamping a plant for high pressure melamine synthesis, wherein the plant has a high pressure section which includes essentially a melamine reactor (1) fed with urea melt, and the raw melamine produced inside said reactor is treated in a quenching section operating at a lower pressure, the method comprising the installation of at least one second stripping reactor (2) and a scrubber (3) in the high pressure section, and wherein the urea melt is fed to the scrubber and subsequently to the melamine reactor (1), the raw melamine is treated inside the second stripping reactor (2), and the gases which emerge from said two reactors are fed to the scrubber for washing and heat exchange with the recirculating urea melt.

## Description

### Field of application

The invention relates to the field of melamine production from urea and relates in particular to a method for revamping a high pressure melamine plant.

### Prior Art

The processes for the synthesis of melamine from urea comprise low pressure catalytic processes and high pressure non-catalytic processes, typically above 7 MPa. These processes are well-known in the art.

A conventional type of plant for the synthesis of melamine using the high pressure non-catalytic process comprises a high pressure section substantially limited to a reactor, called melamine reactor. In said melamine reactor, the urea melt feed is converted into raw melamine with addition of heat, for example from heating bodies supplied with molten salts.

Conversion of urea into melamine also generates gases consisting mainly of ammonia and carbon dioxide (so-called "off-gas") and a number of by-products (mainly OATs and polycondensates). The reaction products (melamine and off-gas), the by-products and the unreacted urea are further processed in a quenching section which operates at significantly lower pressure and temperature. For example, the melamine reactor operates at about 8 MPa and 380 °C, while the quenching section operates at 2-3 MPa and about 160 °C.

In the quenching section, melamine is dissolved in an ammonia aqueous solution and then conveyed to a following purification section; the off-gas are saturated with water and conveyed to a condensation section and then recycled.

Generally, the melamine plant is combined with a urea plant which produces the urea melt feed, starting from NH₃ and CO₂. It is known to recycle the off-gas produced by the melamine plant to the urea plant; for this purpose, typically, the off-gas are condensed with a carbamate solution supplied from a recovery section of the urea plant, resulting in a solution containing NH₃ and CO₂ which is then conveyed back to the urea synthesis loop.

This configuration is widely used, but has a number of drawbacks. The main drawback is that the off-gas are exported at a low pressure and low temperature and with a significant water content due to saturation in the quenching section. These conditions are not optimum for recycling to the urea plant: the low pressure does not allow recycling of the off-gas directly into the urea synthesis section; moreover it is well known that it is not desirable to introduce water into the synthesis loop of a urea plant. Another drawback of this plant configuration is poor conversion of the urea into melamine, which involves greater specific consumption of urea and greater energy consumption in the downstream sections.

### Summary of the invention

The invention aims at revamping the high pressure melamine plants of the abovementioned type, in order to eliminate or reduce the drawbacks discussed above.

The object is achieved by means of a method for revamping a high pressure melamine plant according to the accompanying claims.

The invention relates to revamping of a plant wherein: the high pressure section comprises a melamine reactor fed with urea melt and producing raw melamine; said raw melamine is subjected to depressurization and is treated in a quenching section operating at a pressure substantially lower than that of the high pressure section.

The method according to the invention envisages the installation of at least one second stripping reactor and a scrubber in the high pressure section, and also envisages that:
at least part of the urea melt feed is directed to said scrubber,
the raw melamine flowing out from the melamine reactor is fed to said second stripping reactor, and said second reactor is fed with gaseous ammonia as stripping agent,
at least part of the off-gas collected from said ammonia reactor and/or from said stripping reactor are fed to said scrubber, inside which the off-gas are brought into contact with the urea melt feed.

The term "high pressure section" denotes a set of apparatus which operate substantially at the same nominal pressure (head losses aside). In the revamped plant according to the invention, the melamine reactor, the additional stripping reactor and the scrubber operate substantially at the same nominal operating pressure, said pressure being greater than 7 MPa and preferably greater than 10 MPa, for example 11 MPa.

The scrubber added to the high pressure section has substantially the functions of: preheating the urea melt feed with the heat of the off-gas supplied by the first and/or second reactor; at the same time cooling the off-gas; removing melamine (entrained or sublimated) contained in the off-gas before they are condensed.

The second reactor, termed "stripping reactor", has substantially the functions of: subjecting the raw melamine supplied by the melamine reactor to a stripping process, together with gaseous ammonia as stripping agent and in particular for separating the carbon dioxide contained in the raw melamine; converting the by-products (mainly the polycondensates) into melamine. The purified melamine (melamine melt) obtained in the stripping reactor is conveyed to the quenching and purification sections, which may be kept in the original layout or modified if necessary.

Preferably, the method according to the invention also comprises the installation of a steam generator inside the high pressure section, using the flow of off-gas from the melamine reactor and/or the second stripping reactor as heat source.

Further apparatus added to the high pressure section typically comprise the urea circulation pumps and other auxiliary devices, which are not described in detail.

In one embodiment of the invention, the existing melamine reactor may be retained. In this case, the newly installed apparatus added to the high pressure section operate advantageously at the same pressure as the existing reactor. In another embodiment, the melamine reactor is also revamped or replaced with a new reactor. Revamping or replacement of the melamine reactor may allow raising the operating pressure of the high pressure section.

An important advantage of the invention consists in the fact that the off-gas are produced substantially at the same pressure as the melamine synthesis and at high temperature, and in particular in anhydrous form. This allows (after condensation) recycling to the urea plant of a stream of carbamate which is already at high pressure and temperature, i.e. does not require energy in order to be compressed and/or reheated, and which is water-free, avoiding the drawback of introducing water into the urea synthesis loop. Other advantages are as follows: the possibility of producing steam at a considerable pressure, generally about 0.6 MPa, inside the steam generator added to the high pressure section, owing to the heat of the off-gas; high rate of conversion of urea into melamine; effective removal of the CO₂ dissolved in the raw melamine, owing to the stripping action inside the second reactor.

A further aspect of the invention envisages the installation of an off-gas condensation section, suitable for condensing the high pressure off-gas.

The abovementioned advantages result in: lower specific urea consumption (urea for melamine produced) owing to the improved conversion rate; lower specific steam consumption, mainly owing to the steam produced inside the steam generator of the high pressure section and in the off-gas condenser. The advantages will emerge even more clearly with the aid of the detailed description below relating to a number of preferred embodiments.

### Description of the figures

Fig. 1 is a schematic diagram of the high pressure section of a melamine plant modified according to a preferred embodiment of the invention.
Fig. 2 is a schematic diagram of the off-gas condensation section of the plant according to Fig. 1.

### Detailed description

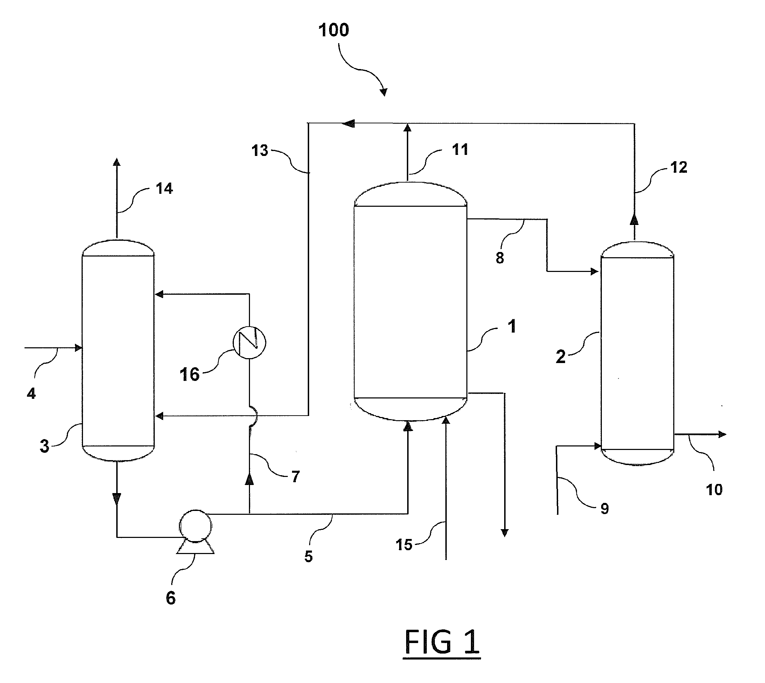

With reference to Fig. 1, the high pressure section 100 of a melamine plant, originally, comprises essentially a melamine reactor 1 fed with urea melt, and the raw melamine produced in said reactor 1 is depressurized and conveyed to a quenching section (not shown).

The high pressure section 100 is modified with the installation of a second stripping reactor 2 and a scrubber 3, and the connection lines shown in the figure.

In particular, the section 100 is modified with: a line for feeding the urea melt 4 to said scrubber 3 rather than directly to the melamine reactor 1; a line for feeding the urea melt 5 leaving the bottom of the scrubber 3 to the melamine reactor 1; a line conveying the raw melamine 8 output from the reactor 1 to the new stripping reactor 2. Moreover, lines are provided for collecting the gases which emerge from the reactors 1 and 2 and conveying them to the scrubber 3, as will be explained below.

The line 5 comprises a urea pump 6 and, advantageously, a line 7 for recirculating part of the urea melt inside the scrubber 3. A heat exchanger 16 is advantageously installed along the recirculation line 7 for cooling the urea, for example producing vapour.

The stripping reactor 2 is further fed with gaseous ammonia 9 which constitutes a stripping agent. The raw melamine, which is fed via the line 8, contacts in counter-flow the gaseous ammonia 9 inside the reactor 2. The purified melamine 10 leaving the stripping reactor 2 is fed to the quenching section of the plant, generally operating at a lower pressure than the section 100.

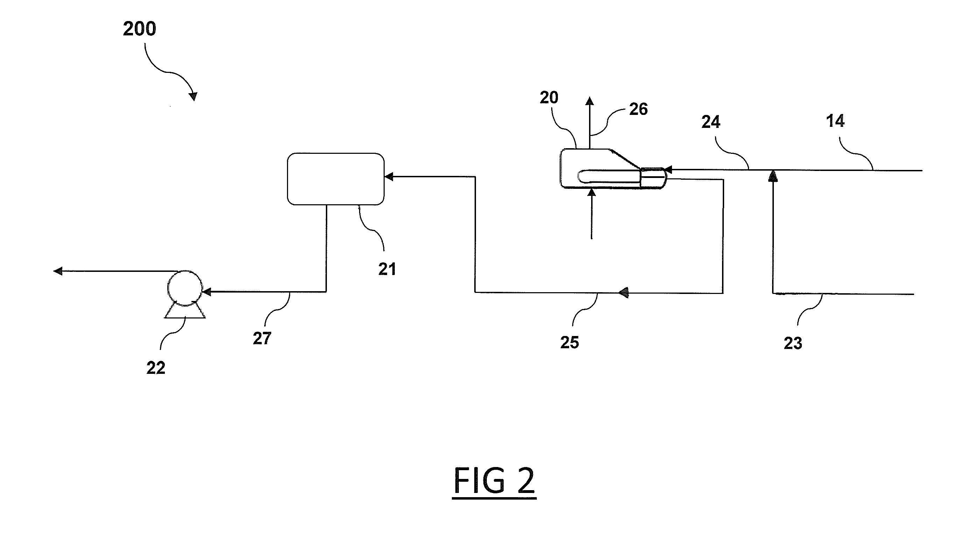

The off-gas streams 11,12 extracted respectively from the melamine reactor 1 and the stripping reactor 2 are combined in a stream 13 which is introduced into the scrubber 3. The stream 12 also contains the gaseous ammonia 9 introduced into the reactor 2 as stripping agent. Inside the scrubber 3, the gaseous stream 13 undergoes washing with the urea melt streams 4 and 7, respectively feeding and recirculating streams. A high pressure stream of anhydrous off-gas 14, mainly composed of ammonia and carbon dioxide, is extracted from the scrubber 3 and is directed to a condensation section, realized for example as shown in Fig. 2.

The line 15 represents a heat-carrier fluid which supplies heat to the melamine reactor 1 in order to promote the endothermic reaction for conversion of urea into melamine. Typically said heat-carrier fluid consists of molten salts which circulate inside suitable heating pipes of the reactor.

In some embodiments the melamine reactor 1 is modified, preferably so as it is able to withstand a higher pressure. For example, reactor 1 may be modified by maintaining the internals and by replacing the shell with a new shell suitable for withstanding a higher pressure. In this way, the method according to the invention may also comprise an increase in the operating pressure of the high pressure section 100. For example, a section originally operating at about 7-8 MPa may be modified so as to operate at a pressure of about 10-11 MPa. In other embodiments, reactor 1 may be replaced by a new reactor.

The urea melt 4 for example is produced in a urea plant connected to the melamine plant comprising the section 100. The off-gas 14, in view of their content of NH₃ and CO₂ (which constitute the reagents for obtaining urea), are preferably recycled to said urea plant.

It should be noted that the stream of off-gas 14 is produced substantially at the operating pressure of the section 100. This other pressure, in addition to the absence of water, represents an important advantage compared to the original configuration. Advantageously, the stream 14 is condensed always at a high pressure, obtaining a recycling solution which may be fed directly to a loop for high-pressure synthesis of the urea.

Fig. 2 shows a preferred embodiment of a condensation section 200 designed for this purpose, i.e. for condensing said stream 14 of high-pressure off-gas.

Said condensation section 200 essentially comprises an off-gas condenser 20 and a carbamate storage tank 21 (also called carbamate receiver) operating in series, and a carbamate pumping section 22.

The anhydrous stream of off-gas 14 is mixed with a small quantity of carbamate solution 23 supplied from the urea plant, generating a two-phase stream 24.

The addition of said carbamate solution 23, upstream of the condenser 20, has the dual function of favouring condensation of the vapours and reducing the crystallization temperature of the carbamate solution.

Said two-phase stream 24 enters the high-pressure off-gas condenser 20, where the condensation of the off-gas takes place, resulting in a solution of condensed carbamate 25. Said solution 25 is conveyed to the tank 21 and the stream 27 thus obtained is injected directly into the synthesis section of the urea plant by means of the pumping section 22. It should be noted that the stream 27 is produced at a high pressure, which is of the same order of magnitude as the pressure of the section 100 (head losses aside), and therefore the cost and energy consumption of the pumping section 22 are reduced thanks to the invention.

The condensation heat of the off-gas is recovered with the production of steam 26 in the shell side of the condenser 20. This steam 26 is subsequently used in the urea or melamine plant, resulting in further energy savings.

## Claims

1. Method for revamping a plant for high pressure melamine synthesis, starting from urea melt, in which:
said plant comprises a high pressure section which includes a melamine reactor (1) fed with urea melt, and a quenching section operating at a lower pressure, and wherein said quenching section receives raw melamine flowing out from said reactor after depressurization,
the method being **characterized by** the following operations:
the installation of at least one second stripping reactor (2) and a scrubber (3) in the high pressure section,
installation of a line for introducing at least a portion of the urea melt feed (4) into said scrubber, and for directing the urea melt collected from said scrubber into the melamine reactor (1),
installation of a line for feeding the raw melamine (8) flowing out from the melamine reactor to said second stripping reactor (2), and said second reactor (2) being also fed with gaseous ammonia (9) as stripping agent,
installation of a line (13) for feeding at least a portion of the off-gas collected by said melamine reactor and/or by said stripping reactor to said scrubber, inside which the off-gas are brought into contact with the urea melt feed.

2. Method according to claim 1, also comprising the installation of a steam generator in the high pressure section, the heat source of said steam generator being the urea heated by the off-gas.

3. Method according to claim 1, also comprising revamping of the melamine reactor (1).

4. Method according to claim 1 or 2, comprising the replacement of the existing melamine reactor (1) with a new melamine reactor.

5. Method according to claim 4, wherein the new melamine reactor operates at a higher pressure than the existing reactor.

6. Method according to any one of the preceding claims, comprising the installation of a line for feeding the urea melt from the scrubber to the melamine reactor and the installation of a line (7) for recirculating into the scrubber part of the urea melt.

7. Method according to any one of the preceding claims, comprising the installation of a line (14) for extraction of anhydrous off-gas from said scrubber (3).

8. Method according to any one of the preceding claims, wherein the melamine reactor (1), the stripping reactor (2) and the scrubber (3) operate at the same nominal operating pressure, said pressure being greater than 7 MPa and preferably greater than 10 MPa.

9. Method according to any one of the preceding claims, also comprising the installation of an off-gas condensation section, adapted to condense an anhydrous stream of off-gas extracted from the high pressure section of the melamine plant, producing a recycling solution at a pressure substantially the same as the operating pressure of said high pressure section of the melamine plant.

10. Method according to claim 9, wherein said condensation section comprises an off-gas condenser and a carbamate storage tank arranged in series, and wherein the stream of off-gas is mixed with a carbamate solution, obtaining a two-phase stream which is fed to said condenser.
